# EUROPEAN PATENT SPECIFICATION

(11) **EP 0 539 505 B1**
(45) Date of publication and mention of the grant of the patent: **08.12.1999**
(21) Application number: 91913988.1
(22) Date of filing: 15.07.1991
(51) Int. Cl.: C07D 213/22

(54) **IMPROVED PROCESSES FOR THE PREPARATION OF 2,2'-BIPYRIDYLS**
VERBESSERTE VERFAHREN ZUR DARSTELLUNG VON 2,2'-BIPYRIDYLEN
PROCEDES AMELIORES POUR LA PREPARATION DE 2,2'-BIPYRIDYLES

(30) Priority: 18.07.1990 US 554804
(43) Date of publication of application: 05.05.1993
(73) Proprietor: ZENECA LIMITED, London W1Y 6LN (GB)
(72) Inventor: GOE, Gerald, L., Greenwood, IN 46142 (US); McGILL, Charles, K., Indianapolis, IN 46220 (US); SHERMAN, Angela, Rappa, Lebanon, IN 46052 (US)
(74) Representative: Waterman, John Richard
(86) International application number: US9104973
(87) International publication number: WO9201674

(56) References cited:
- WO-A-90/08139
- GB-A- 1 377 213
- US-A- 3 053 846
- US-A- 3 173 920
- US-A- 3 210 366
- US-A- 3 822 279
- Chemical Abstracts, volume 94, no. 25, 22 June 1981, (Columbus, Ohio, US), see page 587, abstract 208723m, & ROM, 67302 (COMBINATUL CHIMIC, CRAIOVA) 15 July 1979 (cited in the application)

## Description

### Background of the Invention

The present invention relates to improved processes for the preparation of 2,2'-bipyridyls which are characterized by the novel use of supported elemental nickel catalysts and methods for reactivating and for extending the useful life of the same.

2,2'-Bipyridyl compounds are among the most useful of pyridine derivatives. They have demonstrated significant economic and technological importance as chelating agents and paint additives and in improved coating compositions. See G.M. Badger & W.H.F. Sasse, Adv. Heterocyclic Chem., 2, 179 (1963) and US-A-2,526,718 to G.K. Wheeler. They are also widely used to form bridged quaternary salts, commonly known as Diquats which have important herbicidal properties. See L.A. Summers, "The Bipyridinium Herbicides", Academic Press (1980).

Although other routes to 2,2'-bipyridyls have been reported, one of the more advantageous syntheses has been the action of a Raney nickel metal catalyst on pyridine at elevated temperatures. The importance of this catalyst for other reactions harkens back to the early work of Dr. Murray Raney, who originally described the formation and use of a skeletal nickel prepared by treating a nickel aluminum alloy ("Raney alloy") with a caustic material such as sodium hydroxide to leach out the aluminum metal. The resulting skeletal nickel structure has a high surface area compared to other nickel forms, and has been the catalyst of choice for the preparation of 2,2'-bipyridyls from pyridine bases. A primary focus in this area has in turn been to characterize the most preferred ratio of nickel to aluminum in the Raney alloy used and to develop devices and techniques to optimize the conditions and use of the Raney nickel catalyst once made. See, e.g., US-A-3,822,279 to Joy et al.; Chem. Abstr. Vol. 78, 111132z (1973); US-A-No. 3,053,846 to Varcoe; and Chem. Abstr. Vol. 70, 3770g (1969).

Despite its long-standing predominance, Raney nickel and the processes for its use have also demonstrated significant disadvantages. For example, Raney nickel is a fine powder or a wet sludge which severely complicates its handling and use. Early-on, the 2,2'-bipyridyl reaction was carried out by simply refluxing pyridine on the Raney nickel. It was then discovered that this led to rapid deactivation of the catalyst which has been attributed by some to this prolonged contact with the 2,2'-bipyridyl product itself. See Badger & Sasse, supra, p. 199 which reports that "[t]he formation of 2,2'-bipyridine ceases after 50 hours of refluxing." and US-A-3,053,846 to Varcoe. In later work, alternative reaction schemes and equipment were developed, often focusing on some means whereby pyridine vapors are condensed over and then the resulting hot liquid allowed to stand in contact with the Raney nickel catalyst and slowly filter through the bed in a fashion somewhat similar to a common Soxhlet extractor. This method was thought an improvement because the newly condensing pyridine liquid was intended to displace the previous reaction products in hopes of shortening the period over which the catalyst remains in contact with the 2,2'-bipyridyl formed. Compare, US-A-3,053,846 which reports a recovery of 0.0231g of 2,2'-bipyridyl/g Ni/h against W.H.F. Sasse, Org. Syn. Coll., 5, p. 102 (1973) which reports only 0.007g of 2,2'-bipyridyl produced per g of nickel catalyst per hour. However, while such later processes are feasible, their equipment is often difficult and expensive to build and operate especially when attempting to scale up from the laboratory to the industrial level. Additionally, notwithstanding the many efforts to optimize the use of Raney nickel catalyst, such reactions have demonstrated relatively low rates of conversion to 2,2'-bipyridyls.

In addition to these difficulties, Raney nickel with its skeletal structure is well known to be extremely pyrophoric which presents significant other problems in processing and handling. See R. Habermehl, Chem. Eng. Progress, Feb. 1988, pp. 16-19. Moreover, the caustic substances used to treat Raney alloy in forming the catalyst are commonly aqueous solutions. The Raney nickel is thereby formed as an aqueous paste from which the water must be removed for use in this and many other reactions. This step has proven to be particularly dangerous and expensive, and has been the subject of several patents in the field. For example, the Sasse article in Org. Syn. Coll., 5, reported drying Raney nickel catalyst at 25-30°C under partial vacuum (17-20mm) over 4-12 hours while emphasizing caution and noting that when heated under vacuum, Raney nickel catalyst may suddenly give off large quantities of heat and hydrogen resulting in a dangerous explosion. See also, US-A-3,152,137 to Lang et al. (and corresponding GB-A-899,015); and GB-A-948,956. The production of Raney alloy itself also involves a costly process requiring extreme conditions, see US-A-3,822,279 to Joy et al., and disposal of spent Raney nickel catalyst is complicated because it has been identified as a carcinogen. See A. Agoos, Chemical Week, Dec. 10, 1986, pp. 44-47.

Another prevalent problem has been that Raney nickel catalyst tends to suffer a significant loss of activity as the reaction proceeds. Those skilled in this field have yet to discover an effective way to reactivate such a catalyst once it has lost part or all of its activity. One method reported to have some success is to wash the spent Raney nickel with an alcoholic solution of an alkali metal hydroxide such as KOH. See GB-A-1,202,711. Still further, although not addressing reactivation, the initial activation of such pyrophoric catalysts was generally discussed in US-A-3,560,404 to Jung et al. which introduced the catalyst into a non-aqueous organic liquid and in contact with reducing agents such as hydrazine, borohydrides, or hypophosphites until the development of gaseous hydrogen occurred.

The mechanism of this deactivation of Raney nickel through use is not well understood, although as previously discussed some attribute it to prolonged contact of the catalyst with the 2,2'-bipyridyl product. Still other speculation has been that deactivation may be attributed to oxidation of the Raney nickel itself. In any event, deactivation of such catalysts has been a major concern in the field.

Although this background has concentrated thus far on Raney nickel, other types of catalysts have also been reported in the art for the formation of 2,2'-bipyridyls from pyridine bases. Contrary to any perception that oxidation of metal sites deactivates such catalysts, it is interesting to note that GB-A-1,377,213 reported the use of various metal oxides including nickel oxide in the formation of 2,2'-bipyridyls. However, the rates of formation for such 2,2'-bipyridyls were low even when the oxides were supported on an inert support and subjected to temperatures and pressures as high as 340°C and 800 psig. Similarly extreme conditions were used by two researchers who reported obtaining 2,2'-bipyridyl by heating pyridine in the presence of a nickel-alumina catalyst in an autoclave at 320°C to 325°C and at pressures of 42 to 44 atmospheres. See J.P. Wibaut and H.D. Tjeenk Willink, "A Method of Synthesis of 2,2'-Dipyridyl by Catalytic Dehydrogenation of Pyridine Under Pressure," Recuell des Travaux Chimiques des Pays-Bas, Vol. 50, 1931, pp. 287-290. Still other reported catalysts include: Ziegler catalysts (US-A-3,697,534 to Waddan et al.); salts of certain noble metals (GB-A-1,014,076 and Badger & Sasse, Adv. Heterocyclic Chem. 2 (1963)); mixtures of nickel and aluminum (Chem. Abstr. Vol. 94, 208723m (1981) and Chem Abstr. Vol. 99, 139784w (1983)); a Grignard reagent (GB-A-1,060,661); and certain metal or metal alloy bodies impregnated with an alkaline solution of reducing agent (GB-A-1,009,895). All of these have been reported with varying low levels of success.

Further processes for making 2,2'-bipyridyls are described in US-A-3173920, US-A-3210366 and WO-A-9008139.

It is in light of this extensive background that the applicants entered their study in an attempt to discover an improved process and catalyst for the preparation of 2,2'-bipyridyls which are more convenient and effective from all aspects than the prior art Raney nickel and other processes.

### Summary of the Invention

The applicants' invention addresses the various problems of the prior art and provides commercially significant processes using specific catalysts for the preparation of 2,2'-bipyridyls which avoid the problems inherent with Raney nickel catalysts and at the same time achieve substantial technological and commercial advantages. There is therefore provided a process for the preparation of a 2,2'-bipyridyl optionally substituted by 2- or 4-methyl, the process comprising the step of reacting pyridine or 2- or 4-methylpyridine, in the presence of a nickel catalyst wherein the nickel is bound on a suitable support, characterised in that the catalyst has a specific nickel surface area of at least 100m2/g Ni and at least 85% by weight of the nickel present on the support is in its elemental state; and in that the reaction is conducted at a temperature of 175°C to less 200°C at a pressure sufficient to maintain at least some of the pyridine or 2- or 4-methylpyridine in a liquid state during the reaction, and in the presence of an activating solution containing a methanolic solution of a borohydride salt and ammonium hydroxide.

Pressures about equal to or greater than the autogenous pressure of the base at the temperatures of the reaction have been preferred, depending upon the reactor design being used. Suitable supports have included silica, alumina, kieselguhr, and blends of silica and alumina, with alumina alone being most preferred. Methods for forming the catalysts have included known techniques such as impregnation, precipitation and co-precipitation of a nickel salt on or with the selected support material. Once formed, the supported catalysts have been stable highly porous particles of varying shapes such as pellets, spheres, extrusions and tablets and ranging in size generally from about 1/16 - 1/4 inch (1.6 mm to 6.4 mm) in diameter. The catalysts tested have also varied in nickel loading, with most preferred being about 50-60% nickel on alumina as found in catalysts commercially available from the Calsicat Division of Mallinckrodt, Inc. under the series name "Calsicat". Of this nickel present, catalysts with higher amounts exceeding 75% and approaching 100% by weight of the nickel in its elemental, activated state have produced the best results.

In other embodiments, the applicants' invention has to date favored two specific routes to preparing 2,2'-bipyridyls in the improved processes outlined above. The first is a liquid phase reaction involving pumping a stream of pyridine base through a standard hot tube reactor. The second is a catalytic distillation-type reaction using a pressure still having a column containing an amount of catalyst and the pyridine base introduced into the column, but with the 2,2'-bipyridyl product taken off the bottom of the still. In each of these reactions, the applicants have also discovered effective methods for extending catalyst life and activity. In the tube reactor, an amount of a methanolic solution of sodium borohydride and ammonium hydroxide was included in the pyridine feed which improved and extended the activity of the supported elemental nickel catalyst. The catalysts employed in this embodiment comprise a suitable support having bound thereon nickel at least 85% by weight thereof are elemental nickel. Further, the nickel is characterized by having a high specific nickel surface area of at a least 100 m²/g Ni. The lower temperatures at which effective 2,2'-bipyridyl production can be achieved provide advantages, especially on a commercial scale.

In all cases, the applicants' discoveries avoid many of the mechanical and chemical disadvantages encountered with Raney nickel and other prior art processes by utilizing a catalyst which is more conveniently handled, provides improved rates of 2,2'-bipyridyl formation, and is particularly amenable to extended catalyst life and activity and to scaled-up commercial applications.

Related objects and variations as to the detailed aspects of the invention will become apparent from the following description of the preferred embodiment.

### Description of the Preferred Embodiments

For the purposes of promoting an understanding of the principles of the invention, reference will now be made to various embodiments and specific language will be used to describe the same. Preferred have been catalysts in which the nonoxidized, elemental nickel present approaches about 100% by weight of the total nickel component, and pressures about equal to or greater than the autogenous pressure of the base at the temperature of the reaction.

Referring now to the catalysts themselves, the support upon which the elemental nickel is dispersed can be one of many suitable heterogeneous supports known in the art which are capable of withstanding these reaction conditions. For example, common supports such as silica, alumina, kieselguhr, and blends of silica and alumina are suitable for the applicants' processes as described herein. Methods for forming the catalysts can include one of several known techniques such as impregnation, precipitation and co-precipitation of a nickel salt on or with the selected support material. Other characteristics such as the size and shape of the support are influenced by many factors including the amount of catalyst surface area desired, the ease and method of handling proposed, desired flow characteristics, and the like. To date, the applicants have effectively used supports shaped as pellets, spheres, extrusions and tablets and having sizes ranging generally from 1/16-1/4 inch (1.6 - 6.4 mm) in diameter. In any case, the choice of a support, including its size and shape, for use in a particular reaction is both within the skill of those in the art and within the scope of the invention herein.

The applicants have also investigated catalysts having varying degrees of nickel loading on the supports. The catalysts tested thus far in this embodiment have varied from about 30-60% nickel by weight, with the remaining portion being attributable to the support. These catalysts have performed effectively and have been easily prepared by known procedures such as impregnation of the support with a suitable nickel salt such as a nitrate followed by calcination and reduction. See I. Chen and D. Shiue, "Reduction of Nickel-Alumina Catalysts," Ind. Eng. Chem. Res., 27, 429-434 (1988); and I. Chen, S. Lin, and D. Shiue, "Calcination of Nickel/Alumina Catalysts, Ind. Eng. Chem. Res., 27, 926-929 (1988). Other suitable means, such as by precipitation and co-precipitation, are also known in the art as are many commercially available catalysts from sources such as the Calsicat Division mentioned earlier. From this testing, no minimum threshold or maximum loading of nickel on the support has been shown to exist. It can be said, however, that increased nickel loading has generally produced increased product yields, with the applicants' preferred range of loading thus far being about 50-60% nickel by weight. One exception to this general observation has been where the catalyst is one such as the HTC-400® catalyst discussed in a further preferred embodiment below.

Regarding the state of nickel on the support, the applicants have studied catalysts in which a wide range of the nickel present is in its elemental, activated state. It has generally been found that increasing the elemental nickel content has yielded better results, but without any minimum or threshold level being shown. For that reason, it is considered that any catalyst comprising a significant amount of elemental nickel dispersed or otherwise bound on a suitable, high-surface area support will work in the applicants' process. In this regard, the term "significant" is meant to include an amount of elemental nickel present on the support that is effective in achieving improved rates of 2,2'-bipyridyl production such as those expressed in the specific Examples below. The elemental nickel content should be at least 85% by weight of the total nickel component. Particularly with a commercially available catalyst having some initial degree of activation, such as those identified in the paragraph to follow, the applicants have at times increased this activity by an initial pretreatment through heating in a stream of hydrogen or by pretreatment with a sodium borohydride/ammonium hydroxicle/methanol solution similar to that described in M. Scaros, H. Dryden, J. Westrich, O. Goodmonson and J. Pilney, "Activation of a Commercially Available Nickel on Alumina Catalyst," Catalysis of Organic Reactions, Ed. P. Rylander, I1th Org. Reaction Cat. Soc. (1988). The extent of this pretreatment would then determine the final percentage of elemental nickel present on the catalyst.

In this regard, testing to date has shown one catalyst of choice to be HTC-400® catalysts described below. To accomplish the preferred reactions, pressures have been maintained in the reaction vessel sufficient to maintain at least some of the pyridine base in a liquid state during the reaction. More preferably, the reactions have been conducted under pressures about at or above the autogenous pressure of the pyridine base at the reaction temperatures used in order to more substantially maintain its liquid state depending on the procedure used.

Referring now to two specific routes to 2,2'-bipyridyl production which have been favored by the applicants, the first involves a tube reactor through which a stream of pyridine base is pumped preferably in its liquid phase while the second involves a catalytic distillation-type reaction. In the former, 2,2'-bipyridyls have been prepared in effective yields by pumping a liquid stream of pyridine base through a tube reactor which has been packed with an amount of the applicants' supported elemental nickel catalyst as defined above. The tube reactor used thus far has consisted generally of a 4 inch length of 3/4 inch diameter stainless steel tubing (0.065 inch wall thickness, 0.62 inch inner diameter, and 19.8 cm³ volume) provided at each end with a Swagelok cap. Once filled, the reactor was immersed in a standard commercially-available hot sand bath, and pyridine base was pumped through the tube at the desired rate using a conventional low-volume, high pressure piston pump. Although many others equally or more suitable exist, applicants to date have used an Eldex A-30-S pump available from Eldex Laboratories, Inc. of San Carlos, California. This pump was connected to the reactor via standard 1/8 inch stainless steel tubing. A back-pressure regulator was used to control the pressure of the pyridine within the reactor. The reaction mixture exited the reactor through standard 1/8 inch stainless steel tubing, passed through an air-cooled product condenser and was collected in Whitey stainless steel sample cylinders. Temperatures and pressures have been maintained in the tube reactor consistent with those indicated above. Effective flow rates were varied from about 40-500 g pyridine base/hr with the above reactor, which equates to a space/time velocity from about 2-100 g pyridine base/ml catalyst/hr. This high-volume/low net approach may be advantageous in many situations when comparing effective product yields over a certain quantity of catalyst and time period. This relationship may be expressed as grams of 2,2'-bipyridyl produced per gram of nickel catalyst per hour of reaction. A shorthand version of this measurement used in the Examples below is expressed as "g/g/h".

Other considerations such as increased catalyst life and decreased rates of fouling, ease of recycling, equipment capabilities and the like may also lead those skilled in the art to operate under differing flow rates or other conditions in order to maximize the benefits achieved in practicing the applicants' process. For example, the unfractionated product outflow has preferably not been recycled back through the reactor because the applicants' work has suggested that this leads to the more rapid inactivation of catalyst. Instead, this reaction mixture has been collected and the 2,2'-bipyridyl removed using conventional distillation techniques before recycling the unreacted base.

In another aspect of the invention, it has been discovered that the pyridine base feed can be spiked with an extremely small amount of a methanolic solution of sodium borohydride and ammonium hydroxide to both activate and extend the useful life of the applicants' catalyst without leading to a significant increase in by-product formation. The preferred spiking or additive solution was prepared by adding 12.6ml of a 29% ammonium hydroxide solution to 40ml of methanol and dissolving 0.6 grams of sodium borohydride in this mixture. Effective results have been observed when the feed stream was spiked with only 0.1-0.2% by weight of this additive solution in respective runs, as is specifically noted in the Examples below. Optimizing the preparation, amount and use of such an additive solution for a given reaction is within the skill of the art, as is the selection and use of other possible spiking agents.

As already stated, the applicants' second favored route is a catalytic distillation-type procedure. In general, a catalytic distillation involves a process and apparatus wherein both vapor and liquid may be brought in contact with the catalyst held in a column bed with the reaction products being simultaneously separated by fractional distillation from the resulting lower- and higher-boiling materials. Further descriptions of such reactions and the equipment employed are found in US-A-4,336,407 to Smith and in articles by J.D. Shoemaker et al., "Cumene by Catalytic Distillation," Hydrocarbon Processing (June 1987) and W.P. Stadig, "Catalytic Distillation," Chemical Processing (Feb. 1987).

To conduct their catalytic distillation-type reactions, the applicants fabricated a conventional pressure still with a column prepared from a 3.5 foot length of stainless steel pipe having a 1.6 inch inside diameter. The column was packed with approximately 1400 grams of catalyst and was set up to operate under increased pressure with a back-pressure regulator used to control the same. The still was also arranged to be operated in batch or continuous modes using common and known techniques. The temperatures and pressures of the reactions were as previously set forth, with the pyridine base preferably just boiling at the conditions selected. For example, for pyridine to boil at a temperature of 220°C, the pressure must be no greater than approximately 133 psig. Under these conditions, a small stream of light ends comprising mostly pyridine with some piperidine and water was taken off the column head at reflux and hydrogen was vented via the back-pressure regulator to maintain the desired pressure. In the batch mode, the distillation still was preferably charged with an amount of pyridine and the reaction allowed to proceed for a set period of time. Thereafter, the product-enriched reaction mixture (containing from 18-27% by weight 2,2'-bipyridyl) was removed from the bottom of the still and the 2,2'-bipyridyl was recovered using a conventional fractionating column. In the continuous mode, a high reflux ratio was preferably maintained while liquid pyridine was fed into the side of the column into the catalyst bed at about the same rate that the product-enriched reaction mixture was removed from the bottom of the still. Alternatively, a separate fractionating column was established as part of the apparatus to continuously recover the 2,2'-bipyridyl from the product mixture and return the unconverted pyridine base to the reaction zone.

As with the tube reaction, the applicants have discovered that a very small amount of spiking additive can also be used to activate and extend the useful life of their catalyst in this catalytic distillation reaction. To accomplish this, for example, an amount of ammonium hydroxide was combined with the pyridine base fed into the column while hydrogen gas was passed up through the catalyst bed with the refluxing pyridine vapor. Although the amounts used may vary greatly, effective results have been observed with a feed containing only about 0.05% by weight concentrated ammonium hydroxide solution and hydrogen gas bubbled at a rate of only 6-10 cc/min as measured at the operating pressure of the still. Using this procedure, the applicants have found that catalyst life has more than doubled in many cases before the need for separate reactivation. The applicants have further found that the catalyst, once reactivated as described below, has performed effectively for almost twice as long as the same type of catalyst which was not treated in this manner. These results are more completely discussed in the Examples below.

In yet another aspect of the invention, it has been discovered that the deactivated catalysts can be effectively restored to a large fraction of their former activities by treating them in situ or separately with increased amounts of the same methanolic solution of sodium borohydride and ammonium hydroxide discussed previously in connection with spiking the feed stream to the applicants' tube reactor. More particularly, this reactivation treatment has involved an additive solution prepared by combining the amounts of these ingredients previously described which result in a solution comprising about 1.3% by weight sodium borohydride, about 28.0% by weight ammonium hydroxide and about 70.7% by weight methanol. The restoration treatment using this solution is further discussed in the Examples below.

The preferred catalyst for the process is HTC-400® a nickel-on-alumina catalyst which is available from Crosfield Catalysts of Warrington, England. This catalyst contains approximately 15% nickel, and has a very high specific elemental nickel area of about 152m²/g Ni. In yet other aspects, this HTC-400® catalyst has a pore volume of 0.37 cm³/g, a bulk density of 0.83 g/cm², a nickel crystallite size of 2.4 nm, a mean pore radius of 71.0 A, a total nickel surface area of 27 m²/g of catalyst, and a degree of reduction from the manufacturer of 85%. Product literature on this HTC-400® catalyst and a family of HTC catalysts is available from Crosfield Catalysts.

In further aspects of this embodiment, preferred process temperatures are from 190° to less than 200°C. Further preferred aspects and parameters of this embodiment are similar to those of the embodiments discussed above, including two additional specific routes to 2,2'-bipyridyls as discussed above (e.g. tube reactor and catalytic distillation processes) but employing the unique catalysts of this embodiment.

### EXAMPLE 1

### Tube Reactor Construction

A standard liquid-phase tube reactor for the applicants' process was constructed using a 4-inch length of 3/4-inch stainless steel tubing capped on each end with Swagelok caps. The reactor was immersed in a Techne fluidized sand bath filled with alundum and a Barber-Coleman Limitrol controller was used to regulate the sand bath temperature. The pyridine base was pumped through the reactor tube at the desired flow rate using a 250 ml positive displacement single stroke piston pump, or the Eldex A-30-S previously described. The pyridine base entered the pump through a Teflon tube with a 5 micron stainless steel filter on the end and exited the pump through 1/16-inch stainless steel tubing. This 1/16-inch tubing was connected to separate 1/8-inch stainless steel tubing which led on to the reactor. A back-pressure regulator was used to control the pressure of the feed stream within the reactor which had a capacity of about 19.8 ml and was packed with the desired catalyst material. The product-enriched reaction mixture then exited the reactor through 1/8-inch tubing and was collected in 75 ml capacity Whitey collection cylinders from which fractions could be taken and various components analyzed.

### EXAMPLE 2

### High Specific Nickel Surface Area Catalysts and Processes

### HTC-400® nickel-on-alumina catalyst from Crosfield

Catalyst was used for this study. This HTC-400® catalyst is about 15% by weight nickel. The catalyst was initially activated according to the method of Scaros et al., supra, with a methanolic solution of sodium borohydride (0.6 g) and ammonium hydroxide (12.6 ml of a 29% aqueous solution). The catalyst was rinsed with distilled water and packed into the reactor of Example 1. The feed to the reactor was pyridine (80 g/hour), spiked with approximately 0.1 % of a solution of similar composition to the methanolic solution used for the initial activation. The reaction temperature was maintained at about 190°C with the back-pressure regulator set at 220 psig. Under these conditions, conversion of pyridine was 0.1176 g/g/h over 10 hours of operation, and a 0.0784 9/9/h obtains over 32 hours of operation.

Additional experiments were successfully carried out under similar conditions, with the temperature of the reactor varying from about 180° to about 220°C. For example, at 200°C, over 32.1/hours of operation (on approximately 6h/day basis) the average conversion (g 2,2 bipyridine/g catalyst/hour) was 0.1130 g/g/h. At 180°C, conversion over a similar time is about 0.05 g/g/h. The significance of these conversions is enhanced because it is calculated on total weight of catalyst packed to the reactor, not on the total weight of Ni present in the catalyst, and further because the HTC-400 catalyst used contains approximately 27% by weight of the amount of nickel contained in Calsicat E-230 TR catalyst used in WO-A-9008139.

## Claims

1. A process for the preparation of a 2,2'-bipyridyl optionally substituted by 2- or 4-methyl, the process comprising the step of reacting pyridine or 2- or 4-methylpyridine, in the presence of a nickel catalyst wherein the nickel is bound on a suitable support, characterised in that the catalyst has a specific nickel surface area of at least 100m²/g Ni and at least 85% by weight of the nickel present on the support is in its elemental state; and in that the reaction is conducted at a temperature of 175°C to less than 200°C at a pressure sufficient to maintain at least some of the pyridine or 2- or 4-methylpyridine in a liquid state during the reaction, and in the presence of an activating solution containing a methanolic solution of a borohydride salt and ammonium hydroxide.

2. A process as claimed in claim 1, wherein the catalyst has a specific nickel surface area of at least 150m²/g Ni.

3. A process as claimed in claim 2, wherein the catalyst is HTC-400® nickel-on-alumina, which contains approximately 15% Ni with a specific nickel surface area of about 152 m²/g.

4. A process as claimed in any one of claims 1 to 3, wherein the reaction is conducted at a pressure equal to or greater than the autogenous pressure of the pyridine or 2- or 4-methylpyridine at the temperature of the reaction.

5. A process as claimed in any one of claims 1 to 4, wherein the catalyst in the shape of pellets, spheres, extrusions or tablets in a size ranging generally from 1/16 to 1/4 of an inch (1.6mm to 6.4mm) in diameter.

6. A process as claimed in any one of claims 1 to 5, additionally comprising the step of treating the catalyst before or after the reaction, with an activating solution containing a borohydride salt and ammonium hydroxide.

7. A process as claimed in any one of claims 1 to 6, wherein pyridine is converted to 2,2'-bipyridyl.

8. A process as claimed in any one of claims 1 to 7, wherein the reaction takes place at a temperature of 190°C to less than 200°C.

9. A process as claimed in any one of claims 1 to 8, wherein the reaction is conducted in a liquid phase tube reactor and additionally comprises the steps of charging the tube reactor with an amount of catalyst, bringing the charge reactor to the temperature and pressure of the reaction and feeding a stream of pyridine or 2- or 4-methylpyridine therethrough at a flow rate of 2 to 100g pyridine or 2-or 4-methylpyridine per ml catalyst per hour.

10. A process as claimed in any one of claims 1 to 8, wherein the reaction takes place in a catalytic distillation reactor and additionally comprises the steps of charging a distillation column with an amount of catalyst, bringing the column to the temperature and pressure of the reaction and introducing a feed of pyridine or 2- or 4-methylpyridine.

11. A process as claimed in claim 10, wherein fresh pyridine or 2- or 4-methylpyridine is supplied to the column before reaction and the 2,2'-bipyridyl or methyl-substituted 2,2'-bipyridyl formed in the catalyst bed is taken off the bottom of the column in the product-enriched reaction mixture.

12. A process as claimed in any one of claims 1 to 11, wherein 0.1 to 0.2% by weight of the activating solution is added.

13. A process as claimed in any one of claims 1 to 12, additionally comprising the steps of isolating and recovering the 2,2'-bipyridyl or methyl-substituted 2,2'-bipyridyl formed from the product-enriched reaction mixture.

## Patentansprüche

1. Verfahren zur Herstellung eines gegebenenfalls 2- oder 4-Methyl-substituierten 2,2'-Bipyridyls, wobei das Verfahren den Schritt der Reaktion von Pyridin oder 2-oder 4-Methylpyridin in Gegenwart eines Nickel-Katalysators umfaßt, in dem das Nickel an einen geeigneten Träger gebunden ist,
dadurch gekennzeichnet, daß
der Katalysator eine spezifische Nickel-Oberfläche von wenigstens 100 m²/g Ni hat und wenigstens 85 Gew.-% des auf dem Träger vorliegenden Nickels in seinem elementaren Zustand ist; und daß die Reaktion bei einer Temperatur von 175 bis weniger als 200°C bei einem Druck durchgeführt wird, der ausreichend ist, um wenigstens etwas des Pyridins oder 2- oder 4-Methylpyridins im flüssigen Zustand während der Reaktion zu halten, und in Gegenwart einer aktivierenden Lösung, die eine methanolische Lösung aus einem Borhydridsalz und Ammoniumhydroxid enthält.

2. Verfahren gemäß Anspruch 1, worin der Katalysator eine spezifische Nickel-Oberfläche von wenigstens 150 m²/g Ni hat.

3. Verfahren gemäß Anspruch 2, worin der Katalysator HTC-400®-Nickel-auf-Aluminiumoxid ist, das ca. 15 % Ni mit einer spezifischen Nickel-Oberfläche von ca. 152 m²/g enthält.

4. Verfahren gemäß einem der Ansprüche 1 bis 3, worin die Reaktion bei einem Druck gleich oder größer als der autogene Druck des Pyridins oder 2- oder 4-Methylpyridins bei der Reaktionstemperatur durchgeführt wird.

5. Verfahren gemäß einem der Ansprüche 1 bis 4, worin der Katalysator in Form von Pellets, Kugeln, Extrusionen oder Tabletten in einer Größe im Bereich von allgemein 1/16 bis 1/14 Zoll (1,6 bis 6,4 mm) Durchmesser ist.

6. Verfahren gemäß einem der Ansprüche 1 bis 5, das zusätzlich den Schritt der Behandlung des Katalysators vor oder nach der Reaktion mit einer aktivierenden Lösung umfaßt, die ein Borhydridsalz und Ammoniumhydroxid enthält.

7. Verfahren gemäß einem der Ansprüche 1 bis 6, worin Pyridin zu 2,2'-Bipyridyl konvertiert wird.

8. Verfahren gemäß einem der Ansprüche 1 bis 7, worin die Reaktion bei einer Temperatur von 190 bis weniger als 200°C stattfindet.

9. Verfahren gemäß einem der Ansprüche 1 bis 8, worin die Reaktion in einem Flüssigphasen-Rohrreaktor durchgeführt wird und zusätzlich die Schritte des Füllens des Rohrreaktors mit einer Menge an Katalysator, das Bringen des gefüllten Reaktors auf die Temperatur und den Druck der Reaktion und das Zuführen eines Stroms an Pyridin oder 2- oder 4-Methylpyridin hindurch mit einer Fließgeschwindigkeit von 2 bis 100 g Pyridin oder 2- oder 4-Methylpyridin pro ml Katalysator pro Stunde umfaßt.

10. Verfahren gemäß einem der Ansprüche 1 bis 8, worin die Reaktion in einem katalytischen Destillationsreaktor stattfindet und zusätzlich die Schritte des Befüllens einer Destillationssäule mit einer Menge an Katalysator, des Bringens der Säule auf die Temperatur und den Druck der Reaktion und des Einführens einer Zufuhr von Pyridin oder 2- oder 4-Methylpyridin umfaßt.

11. Verfahren gemäß Anspruch 10, worin frisches Pyridin oder 2- oder 4-Methylpyridin der Säule vor der Reaktion zugeführt wird und das im Katalysatorbett gebildete 2,2'-Bipyridyl oder Methyl-substituierte 2,2'-Bipyridyl am Sumpf der Säule in der mit Produkt angereicherten Reaktionsmischung abgezogen wird.

12. Verfahren gemäß einem der Ansprüche 1 bis 11, worin 0,1 bis 0,2 Gew.-% der aktivierenden Lösung hinzugegeben wird.

13. Verfahren gemäß einem der Ansprüche 1 bis 12, das zusätzlich die Schritte der Isolierung und Gewinnung des aus der mit Produkt angereicherten Reaktionsmischung gebildeten 2,2'-Bipyridyls oder Methyl-substituierten 2,2'-Bipyridyls umfaßt.

## Revendications

1. Procédé pour la préparation d'un 2,2'-bipyridyle facultativement substitué avec un groupe 2- ou 4-méthyle, procédé qui comprend l'étape consistant à faire réagir de la pyridine ou de la 2- ou 4-méthylpyridine, en présence d'un catalyseur au nickel dans lequel le nickel est fixé sur un support convenable, caractérisé en ce que le catalyseur a une surface spécifique du nickel d'au moins 100 m²/g de Ni et une quantité d'au moins 85 % en poids du nickel présent sur le support est à l'état élémentaire ; et en ce que la réaction est conduite à une température comprise dans l'intervalle de 175°C à moins de 200°C sous une pression suffisante pour maintenir au moins une certaine quantité de la pyridine ou de la 2- ou 4-méthylpyridine à l'état liquide au cours de la réaction, et en présence d'une solution activatrice contenant une solution méthanolique d'un borohydrure et d'hydroxyde d'ammonium.

2. Procédé suivant la revendication 1, dans lequel le catalyseur a une surface spécifique du nickel d'au moins 150 m²/g de Ni.

3. Procédé suivant la revendication 2, dans lequel le catalyseur consiste en nickel sur alumine HTC-400®, qui contient approximativement 15 % de Ni ayant une surface spécifique du nickel d'environ 152 m²/g.

4. Procédé suivant l'une quelconque des revendications 1 à 3, dans lequel la réaction est conduite à une pression égale ou supérieure à la pression autogène de la pyridine ou de la 2- ou 4-méthylpyridine à la température de réaction.

5. Procédé suivant l'une quelconque des revendications 1 à 4, dans lequel le catalyseur est sous forme de pastilles, de sphères, de pièces extrudées ou de comprimés ayant des dimensions correspondant généralement à un diamètre compris dans l'intervalle de 1/16 inch à 1/4 inch (1,6 mm à 6,4 mm).

6. Procédé suivant l'une quelconque des revendications 1 à 5, comprenant en outre l'étape de traitement du catalyseur, avant ou après la réaction, avec une solution activatrice contenant un borohydrure et de l'hydroxyde d'ammonium.

7. Procédé suivant l'une quelconque des revendications 1 à 6, dans lequel la pyridine est transformée en 2,2'-bipyridyle.

8. Procédé suivant l'une quelconque des revendications 1 à 7, dans lequel la réaction s'effectue à une température comprise dans l'intervalle de 190°C à moins de 200°C.

9. Procédé suivant l'une quelconque des revendications 1 à 8, dans lequel la réaction est conduite dans un réacteur tubulaire à phase liquide et comprend en outre les étapes consistant à introduire dans le réacteur tubulaire une quantité de catalyseur, à porter le réacteur chargé à la température et la pression de la réaction et à introduire un courant de pyridine ou 2- ou 4-méthylpyridine à travers ce réacteur à un débit de 2 à 100 g de pyridine ou 2- ou 4-méthylpyridine par ml de catalyseur et par heure.

10. Procédé suivant l'une quelconque des revendications 1 à 8, dans lequel la réaction s'effectue dans un réacteur de distillation catalytique et comprend en outre les étapes consistant à introduire dans une colonne de distillation une quantité de catalyseur, à amener la colonne à la température et la pression de la réaction et à introduire une charge de pyridine ou 2- ou 4-méthylpyridine.

11. Procédé suivnat la revendication 10, dans lequel la pyridine ou 2- ou 4-méthylpyridine fraîche est introduite dans la colonne avant réaction et le 2,2'-bipyridyle ou 2,2'-bipyridyle à substituant méthyle formé dans le lit de catalyseur est évacué par le fond de la colonne dans le mélange réactionnel enrichi en produit.

12. Procédé suivant l'une quelconque des revendications 1 à 11, dans lequel une quantité de 0,1 à 0,2 % en poids de la solution activatrice est ajoutée.

13. Procédé suivant l'une quelconque des revendications 1 à 12, comprenant en outre l'étape consistant à isoler et recueillir le 2,2'-bipyridyle ou 2,2'-bipyridyle à substituant méthyle formé à partir du mélange réactionnel enrichi en produit.
